# EUROPEAN PATENT SPECIFICATION

(11) **EP 4 304 532 B1**
(45) Date of publication and mention of the grant of the patent: **10.09.2025**
(21) Application number: 22714241.1
(22) Date of filing: 04.03.2022
(51) Int. Cl.: A61F 2/44, A61B 17/88

(54) **CAGE FOR VERTEBRAL ARTHRODESIS OPERATIONS**
KÄFIG FÜR WIRBELARTHRODESEOPERATIONEN
CAGE POUR OPÉRATIONS D'ARTHRODÈSE VERTÉBRALE

(30) Priority: 08.03.2021 IT 202100005372
(43) Date of publication of application: 17.01.2024
(73) Proprietor: Lamartina, Claudio, 20126 Milano (IT); Berjano Coquillat, Pedro Luis, 20145 Milano (IT)
(72) Inventor: Lamartina, Claudio, 20126 Milano (IT); Berjano Coquillat, Pedro Luis, 20145 Milano (IT)
(74) Representative: Raimondi, Margherita
(86) International application number: PCT/IB2022/051933
(87) International publication number: WO 2022/189921

(56) References cited:
- WO-A1-2010/028045
- WO-A1-2011/056172
- WO-A1-2012/008279
- WO-A2-2006/033067
- WO-A2-2008/024664
- US-A1- 2004 158 328
- US-A1- 2014 074 241
- US-A1- 2014 364 917
- US-A1- 2015 057 753
- US-A1- 2016 051 374

## Description

The present invention relates to a cage for vertebral arthrodesis operations and an adapter plate for a cage for arthrodesis operations.

It is known in the technical sector of vertebral pathology that the profile of the vertebral column depends on the form of the intervertebral discs and that this profile is altered, among other things, by the presence of forms of arthrosis, vertebral deformity, trauma or other factors, as a result of which the discs lose their normal form.

It is also known that there exists a technique called vertebral arthrodesis by means of which the fusion (formation of bone bridges) between adjacent vertebrae is surgically induced in order to treat the different vertebral pathologies such as pain originating from the discs, arthrosis, instability, fractures, pseudoarthrosis, tumours; these deformities inducing the said and required variation in profile.

Vertebral arthrodesis is facilitated by the insertion between adjacent vertebrae of so-called cages made of osteo-integrative material or filled with osteo-integrative material suitable for promoting fusion between vertebrae.

It is also known that the final position, in contact with the vertebral endplates of the adjacent vertebrae, assumed by the cage once insertion has been completed is highly influential on the results for the patient, in particular with regard to the restoration of suitable biomechanics of the column and stability of the implant.

In this connection it is pointed out that the insertion of the cages inside the intervertebral discs is generally performed by means of a tapping action and the insertion trajectory of the cage depends on more than factor, including: the starting point of insertion, the direction of the insertion axis and the trajectory where there is less resistance.

A factor of fundamental importance for a correct insertion, however, is the point of entry of the cage inside the discs.

In this connection it is also known that the capacity of the operator to direct the insertion trajectory and therefore the final positioning of the cage is limited owing to the difficulty of controlling the aforementioned factors and in particular the fact that the point of entry of the cage may be different from an ideal position thereof, since conditioned by the presence of anatomical structures such as arteries, veins and nerves situated between the ideal entry point and the entry point which the operator is forced to adopt owing to the said structures which impose a different point of insertion between the vertebral bodies.

The outcome of the operation is therefore rendered difficult from the start owing to the insertion of the cage from an incorrect insertion point.

Similarly, a correct insertion point, but an insertion direction conditioned by the presence of anatomical structures whose position cannot be modified, may result in insertion in an unsuitable final position.

Insertion is generally performed by means of a guide tool, in the form of an elongated longitudinal body, extending between a first end, configured for rigid connection with the pushing side of the implant, and an opposite end on which the operator performs a tapping/hammering action, resulting in linear insertion of the implant with a substantially rectilinear direction of the "pushing" force and insertion trajectory, causing the cage to be displaced along the tapping axis.

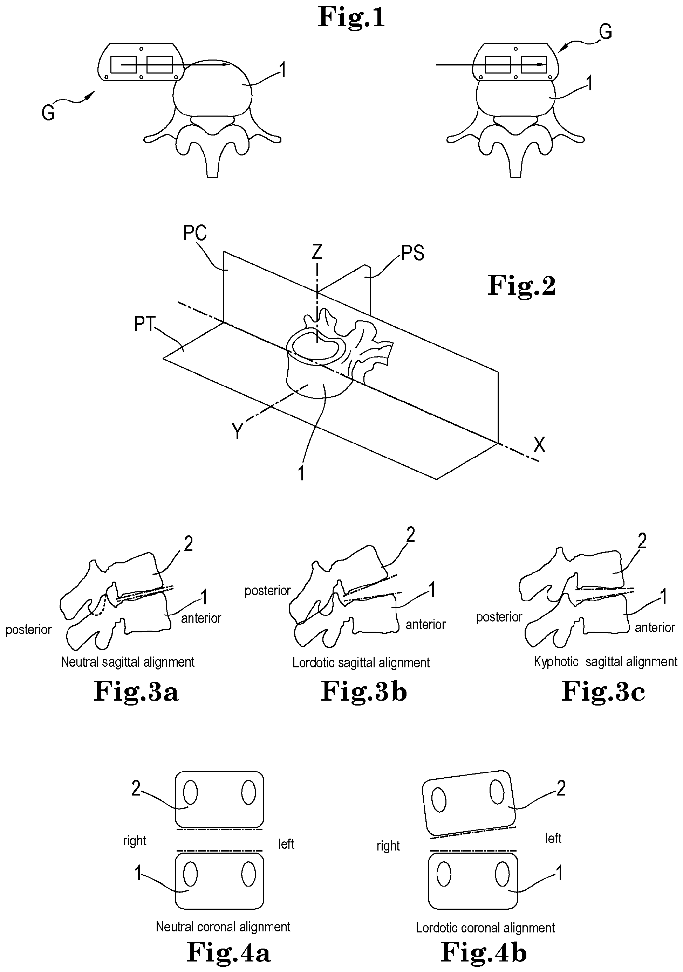

An example according to the prior art where a cage "G" is inserted from an incorrect entry point, resulting in a non-optimal end position, is illustrated in Fig. 1.

WO2011056172 describes an intervertebral implant comprising an insertion end, an end for engagement with an insertion tool, opposite to the insertion end, and a first surface and a second surface situated opposite each other and configured to come into contact with respective adjacent vertebral endplates.

A slot is formed at the engaging end and extends continuously between and at least partially along the front wall and rear wall. A rotating pin is positioned inside the slot and is configured for rotational coupling with an insertion instrument designed to cause a rotation of the implant during insertion.

A plurality of discontinuous curvilinear reliefs, which are interrupted so as to form a plurality of teeth, are arranged on the top surface and bottom surface of the implant.

The teeth of the top and bottom reliefs come into contact with the endplates of the vertebral bodies so as to cause rotation of the implant, produced by rotation of the guide tool, and to guide the insertion trajectory of the implant while the insertion end advances within the disc space. The teeth formed by the interrupted reliefs, are designed to grip the vertebral bodies so as to form grooves and favour the stabilization of the implant.

With this implant and insertion tool it is possible to obtain a certain rotation of the implant during insertion, but they require a tool with a rotatable connection and are not satisfactory if an insertion point is misaligned with respect to the ideal point; furthermore, the teeth bite into the vertebral endplates in order to increase the stabilizing action, with the risk of damaging them.

Further examples of techniques for inserting intervertebral implants, known for example from US 2014-364917 or US2014074241A1, involve the formation of deep grooves in the vertebral endplates, in order to insert, following positioning of the implant, separate stabilization elements which are stably joined to the implant and anchor it to the vertebral bodies.

These known techniques do not solve the problem of an incorrect insertion trajectory or unfavourable entry point and at the same time require deep incision of the vertebral endplates, something which may result in breakage of the vertebral bodies.

The technical problem which is posed, therefore, is that of designing a cage which allows easier and more precise insertion and in particular is suitable, during insertion, for being arranged independently in the correct position between the vertebral bodies and the inside the disc, also in the case of an entry point which is incorrect or in any case different from the ideal entry point or in the case of a non-optimal insertion angle.

In connection with this problem, it is also required that the cage should be able to avoid damage to and breakage of the bone of the vertebral bodies between which it is inserted.

In particular, it is desirable that the implant should not require the prior formation of grooves in the bone and that it should be easy and inexpensive to produce and be able to be easily inserted in the discs using normal standardized insertion means.

These results are obtained according to the present invention by a cage for vertebral arthrodesis treatment according to the characteristic features of Claim 1.

Such a cage has a body comprising a first surface for contact with a first vertebral endplate and a second surface for contact with a second vertebral endplate, the first and second surfaces being arranged opposite each other in a coronal heightwise direction of the cage, and with an entry side for entry into the intervertebral disc space and a pushing side opposite to the entry side in a direction of insertion between the vertebral endplates. The cage also has at least one continuous directional guide relief (or keel) on at least one of the two said opposite surfaces for contact with a vertebral endplate; said guide relief or keel being configured and arranged so as to guide the cage along a predefined movement trajectory suitable for insertion of the cage between the first vertebral endplate and the second vertebral endplate, from an entry point as far as a predefined and correct end position between the first vertebral endplate and the second vertebral endplate, as a result of insertion of the cage between the vertebral endplates from the entry side by means of action on the pushing side.

The present invention relates to a cage and an adapter plate as claimed hereafter. Preferred embodiments of the invention are set forth in the dependent claims.

According to the invention, the cage comprises a continuous relief which has a rectilinear development inclined in the plane of the contact surface or a mixed development with at least one rectilinear section inclined in the plane of the contact surface, designed to cause a gradual deviation of the cage from a rectilinear insertion trajectory.

The at least one directional guide relief is also characterized in that it projects from the contact surface of the cage with a height in the vertical-coronal direction of between 0.5 and 4 mm, preferably less than or equal to 2 mm and in particular of between 1 and 2 mm.

The design and the development of the guide relief or keel result in the division of the pushing force into at least two components, the resultant of which determines a variation in the rectilinear direction of insertion such as to cause a gradual deviation of the entire cage from the rectilinear trajectory, so as to define a modified insertion trajectory from the entry point to a correct end position.

In particular, the design and rectilinear development which is inclined or has an inclined rectilinear section of the keel result in a gradual deviation of the cage from the rectilinear insertion trajectory, adding to the component for displacement of the cage along the tapping axis a displacement component orthogonal to the tapping axis.

Owing to this configuration of the at least one guide relief and to the reduced height, it is therefore possible to obtain the desired insertion trajectory, affecting only and as little as possible the superficial part of the bone of the vertebral endplate without entering substantially into the spongy part of the bone, thus reducing the risk of damage and avoiding fractures of the vertebral body. The inclination of the relief also avoids stressing the spongy part of the bone in the direction parallel to the lamellar structure thereof, further reducing the risk of damage.

These effects are also advantageously obtained without the need to introduce degrees of freedom (rotation) between the guide tool and cage.

The cage according to the present invention is therefore able to ensure correct positioning also in the case of an entry pointy or rectilinear insertion trajectory which is not ideal, without damaging or fracturing the vertebral body.

In a preferred embodiment, a directional guide relief may have a rectilinear development or mixed development with at least one rectilinear section and at least one curvilinear section, each solution being designed to determine corresponding and suitable modifications of the entry trajectory of the cage into the intervertebral space.

Preferably, the mixed profile relief comprises a first inclined rectilinear section, proximal to the entry side, and a second curvilinear section connected to the rectilinear section. The function of the curvilinear section is that of causing a rotation of the cage along the insertion trajectory.

According to further preferred embodiments of the cage according to the invention, the directional guide relief is uninterrupted, extending over the entire surface of the contact surface from the entry side to the pushing side; or else the directional guide relief extends over a partial section from the entry side to the pushing side. By configuring a relief with different lengths and angles of inclination it is possible to obtain different modifications of the entry trajectory.

It is also envisaged that the opposite surfaces of the cage according to the invention have an inclination increasing from the entry side to the pushing side.

According to preferred embodiments it is envisaged that the cage has a transverse dimension greater than the coronal dimension for latero-lateral insertion or a transverse dimension congruous with the coronal dimension for antero-posterior insertion.

Cages according to the invention may be advantageously designed and configured for anterior, oblique anterior, lateral, oblique lateral, posterior, oblique posterior insertion and/or with rectilinear or curvilinear guided trajectories or combinations thereof.

A further aspect of the present invention relates to an adapter plate for a cage for vertebral arthrodesis operations, which allows conventional cages which do not have guide reliefs to be adapted by providing them with a surface for contact with the vertebral endplate comprising at least one directional guide relief according to the advantageous characteristics illustrated in connection with the cage according to the present invention.

Further details may be obtained from the following description of a nonlimiting example of embodiment of the subject of the present invention provided with reference to the attached drawings in which:
Figure 1: shows a plan view of an implant according to the prior art;
Figure 2: shows a perspective view of the planes and axes of orientation of a vertebra according to the prior art;
Figures 3a-3c: shows a plan view of the orientations which, according to the prior art, adjacent vertebrae may assume with respect to a sagittal plane;
Figures 4a-4b: show a plan view of the orientations which, according to the prior art, adjacent vertebrae may assume with respect to a coronal plane;
Figure 5: shows a plan view of a model of a first example of embodiment of a cage according to the present invention;
Figure 6: shows a perspective view of the cage model according to Fig.5;
Figure 7: shows a plan view of a model of a second example of embodiment of a cage according to the present invention;
Figure 8: shows a plan view of a first example of a cage with partial inclined keel according to the present invention;
Figure 9: shows a perspective view of a model of a second example of a cage according to the present invention;
Figure 10: shows a plan view of an example of embodiment of a cage according to the present invention for lateral insertion;
Figure 11: shows a plan view of a first example of embodiment of a cage according to the present invention for antero-posterior insertion;
Figure 12: shows a plan view of a second example of embodiment of a cage according to the present invention for antero-posterior insertion;
Figure 13: shows a view from above of a further example of embodiment of a cage according to the present invention for lateral insertion with a mixed-profile keel;
Figure 14: shows an exploded perspective view of an example of embodiment of a cage according to the present invention, comprising a first and a second adapter plate according to the present invention;
Figure 15: shows a cross-sectioned view along a coronal-sagittal plane passing along the median line T of the cage according to Fig. 14, in the assembled configuration with adapter plates;
Figure 16: shows views of the sequence for left-hand lateral insertion of a cage according to the invention.

As shown in Fig. 2 and solely for easier understanding of the description of the examples of embodiment of cages according to the present invention, the following planes and axis which may be related to the vertebral bodies 1 and intervertebral discs (not shown) are defined:
- transverse plane PT: parallel to the bottom vertebral endplate of the vertebra situated above the disc and the top vertebral endplate of the vertebra situated below the disc;
- sagittal plane PS: orthogonal to the transverse plane PT and oriented along an antero-posterior direction of the vertebrae;
- coronal plane PC; orthogonal to the transverse plane and oriented along a latero-lateral direction of the vertebra;
- vertical axis Z-Z: orthogonal to the transverse plane;
- sagittal axis Y-Y; contained in the transverse plane and parallel to the antero-posterior axis of the vertebra;
- transverse axis X-X: contained in the transverse plane, oriented along a latero-lateral direction of the vertebra and perpendicular to the sagittal axis.

The following Figures 3 and 4 also show the different conditions which the two adjacent vertebrae 1,2 may have in relation to the sagittal plane (with neutral, lordotic or kyphotic coronal alignment) or coronal plane (with neutral or scoliotic coronal alignment) as a result, among other things, of a deterioration in the state of the intervertebral disc situated between them.

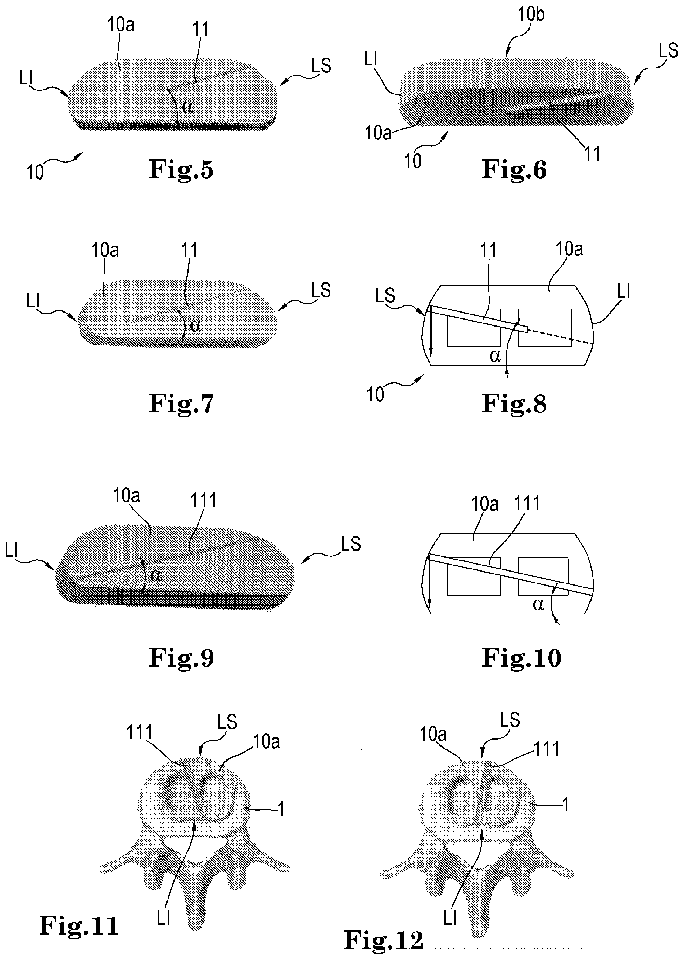

With reference to Figs. 5 and 6, a first example of a cage model for vertebral arthrodesis treatment according to the invention, for which an entry side LI of the cage into the intervertebral space and an opposite pushing side LS of the cage for causing insertion thereof between the vertebrae are defined, comprises a body 10 of suitable thickness in the coronal heightwise direction Z-Z, which has opposite surfaces in the coronal direction Z-Z, i.e. top surface 10a and bottom surface 10b, each suitable for contact with a respective vertebral endplate of a bottom vertebral body 1 and top vertebral body, not shown, between which the cage is inserted.

In the first example shown, the two surfaces 10a, 10b are substantially flat.

The thickness of the cage in the coronal direction Z-Z is smaller than the other two dimensions and proportional to the degree of correction of the profile of the intervertebral space which the implant must provide.

According to preferred embodiments, it is envisaged that the two opposite faces 10a,10b of the cage may be parallel to each other, being particularly suitable for insertion between vertebrae in the neutral sagittal alignment condition (Fig. 3a) and/or neutral coronal alignment condition (Fig. 4a).

According to further preferred embodiments, the opposite faces 10a,10b making contact with the vertebral endplates may have an inclination of at least one of the two relative to the other one equal to an angle of between 5 and 30 degrees from the entry side LI to the pushing side LS; this inclination may be configured so as to correspond to the different conditions of the adjacent vertebrae which, with reference to the sagittal planes, may have lordotic conditions (Fig. 3b) or kyphotic conditions (Fig. 3c); while with reference to the coronal planes they may have scoliotic conditions, which must be suitably treated and corrected.

According to the invention it is envisaged that at least one of the two surfaces 10a or 10b for contact with the adjacent vertebra(e) comprises at least one continuous relief 11, below also referred to as "keel", extending along at least part of the said surface and having a directional guiding function for guiding the cage along a predefined movement trajectory during insertion inside the intervertebral disc, from an entry point as far as a predefined and correct end position between the first vertebral endplate and the second vertebral endplate, when the cage is inserted from the entry side by means of a pushing action, in particular tapping, on the pushing side.

In greater detail, the design and development of the keel is designed to cause the division of the pushing force into two components, the resultant of which is designed to cause a variation in the direction of insertion, so as to modify the insertion trajectory, causing a gradual deviation of the entire cage from the insertion/tapping axis, between the entry point and a correct end position, even in the case where the entry point is not ideal.

In a first embodiment according to the invention, the keel 11 has a rectilinear development with an inclination from the entry side LI to the pushing side LS, at an acute angle α.

As shown in Figs. 9-11, the angle α of inclination may be measured in the plane of the contact surface, with respect to a side or axis of the cage which connects the insertion side to the pushing side.

According to the preferred embodiments of the cage it is envisaged that:
- the keel 11 extends (Figs. 5,6,7,8) only along a part, preferably over between half and two-thirds, of the length in the transverse direction X-X of the cage (partial keel), in which case the part of the contact surface without keel will correspond to its end proximal to the entry side, namely the end which is inserted first of all into the intervertebral space; this configuration is suitable for causing small and/or partial deviations of the insertion trajectory;
- the cage has one or more holes which are designed to favour the vertebral fusion of adjacent vertebral bodies formed in zones which are not occupied by one or more directional keels;
- the keel 111 extends (Figs. 9, 10, 11, 12) over the whole length of the cage (full keel); this solution, for the same angle α, allows a greater correction of the insertion trajectory.

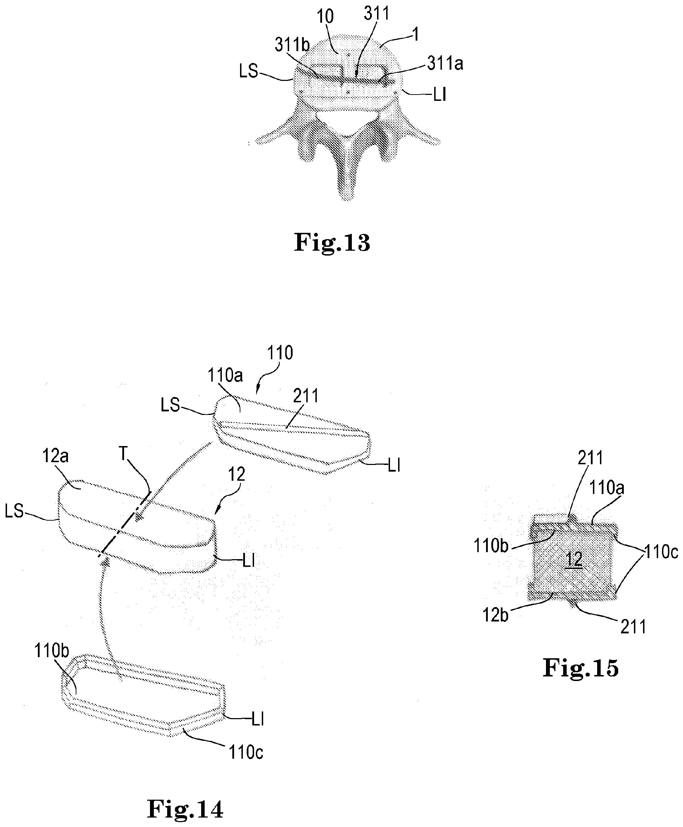

According to preferred embodiments, the keel may have a mixed development (Fig. 13), namely rectilinear development 311a over at least a first section, preferably proximal to the entry side LI, and curvilinear development 311b over a second section connected to the first section.

The function of the section with a curvilinear profile is to cause a rotation of the cage along the insertion trajectory, so as to compensate for any incorrect insertion alignment due to an incorrect initial insertion condition or to anatomical structures which do not allow a linear insertion trajectory.

According to further preferred embodiments, it is envisaged that the cage has a plurality of directional keels parallel to each other. The use of several keels helps reduce the risk of damage to the vertebral endplate.

According to the invention it is envisaged that the guide keel projects from the surface of the cage with a height in the vertical/coronal direction Z-Z of between 0.5 and 4 mm, preferably smaller than or equal to 2 mm and in particular of between 1 and 2 mm. This smaller height of the one or more directional keels is able to ensure the guiding action without significantly affecting the bone of the vertebral body; in particular any action of the keel on the vertebral body is limited to the surface of the vertebral endplate or in any case to the coronal part thereof, without touching the spongy part of the vertebral body which has an extremely fragile lamellar structure. This effect is synergically assisted by the inclined rectilinear profile of the keel which prevents stressing of the spongy part in a direction parallel to the orientation of the lamellae.

According to further preferred embodiments it is envisaged that:
- the guide keel has a cross-section along a coronal plane which is generally triangular, preferably in the form of an equilateral or rectangular triangle, or quadrangular, rectangular or trapezoidal; and/or
- a width at the point of joining to the cage of between 1 and 4 mm and at the point of contact with the vertebra of between 0.5 and 4 mm; and/or
- for lateral insertion cages (Figs. 1-6) the angle α is between 5° and 45°; wider angles corresponding to greater correction of the insertion trajectory; and/or
- for antero-posterior insertion cages (Figs. 11, 12) the angle α between the guide keel and the insertion axis is between 5° and 45°.

According to the invention cages may be provided so as to be suitable for anterior, oblique anterior, lateral, oblique lateral, posterior, oblique posterior insertion and with displaced rectilinear or mixed rectilinear-curvilinear guided trajectories.

In greater detail, with reference to the figures there are:
- examples of cage with a partial or complete keel for lateral insertion from left to right (Figs. 5-7,9) or from right to left (Figs. 8,10, 13);
- examples of cages with a respectively partial or complete keel for antero-posterior insertion (Figs.11,12);
use of either one of the embodiments depending on the actual entry point imposed by the anatomical conditions surrounding the spinal column.

A further aspect of the present invention relates to an adapter plate for a cage for vertebral arthrodesis operations.

With reference to Figs. 14 and 15, the cage may be for example a conventional cage with a body 12 comprising a first surface 12a and a second surface 12b situated opposite each other in a coronal heightwise direction Z-Z of the cage, an entry side LI for entry into the intervertebral disc and a pushing side LS, opposite to the entry side in a direction of insertion into the space of the intervertebral disc, suitable for engagement with a guide tool for receiving an insertion thrusting force, generally by means of tapping along a tapping axis.

As shown in Figs. 14 and 15, an example of an adapter plate 110 according to the present invention comprises: a cage body with a first surface 110a for contact with a first vertebral endplate and a second surface 110b for contact with the cage 12, the first and second surfaces being arranged opposite each other in the vertical-coronal heightwise direction Z-Z of the plate, an entry side LI for entry into the intervertebral disc space and a pushing side LS opposite to the entry side in a direction of insertion into the intervertebral disc space.

The form of the plate body 110 is preferably flat with a length greater than its width, but it may have one or more changes in inclination in the vertical direction Z-Z so as to adapt to the surface of a cage with corresponding changes in inclination.

The first surface 110a of the plate has at least one directional guide relief or keel 211 projecting from it, according to one or more of the advantageous characteristic features illustrated in the present description.

The directional keel 211 is in particular made of the same material as the plate body on the first surface 11a, so as to form a single body with the same plate body. The keel 211 may preferably have a triangular, rectangular or trapezoidal cross-section.

During use, the second surface 110b of the plate, which is suitable for making contact with the cage 12, is joined to the first surface 12a or second surface 12b of the plate; cage 12 and plate 100 are therefore joined together so as to form a single monolithic body before insertion into the intervertebral disc. The joining operation may be performed, for example, by means of welding or bonding or mechanically, or a combination thereof.

As shown in Figs. 14 and 15, means for performing joining to the cage 12 may include one or more edges 110c extending in the coronal direction, from an end edge of the plate body, so as to obtain mechanical fixing of the plate 110 to the cage 12.

Alternatively or in addition, means for joining the plate 110 to the cage may include any system for performing mechanical fixing to the cage or to an opposite plate 110 joined to the opposite surface of the cage 12. Preferred fixing systems are:
- one or more holes with respective fixing screws passing through the plate and engaging with the cage;
- one or more holes with respective fixing screws passing through the plate and cage and engaging with the opposite second adapter plate;
- a relief with hole for screws for performing fixing to the cage or to the counter-lateral plate;
- a clip with seat on the surface of the plate and/or the cage.

Once joining has been performed, the first surface of the plate forms the first surface of the cage for contact with a first vertebral endplate, offering the same directional guiding advantages described above in relation to the preferred examples of embodiment of cages according to the invention.

The plate may cover the whole surface of the cage intended to make contact with the bone, or only a part thereof. In preferred embodiments, the plate body is in particular in the form of a strip with a smaller width than the corresponding width of the cage 12, with an inclined directional guiding relief or with at least an inclined section formed on the first surface of the strip.

The plate may have a smooth or rough surface and is preferably made of osteo-conductive and/or osteo-integrative material.

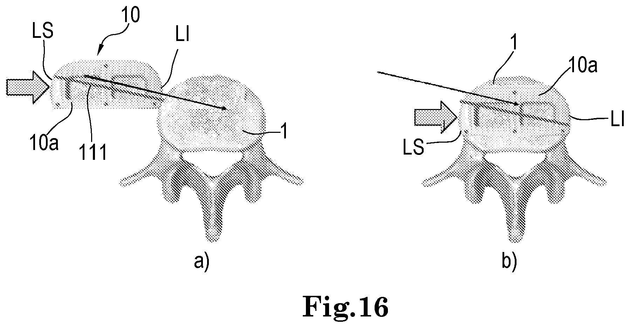

Figs. 16a to 16b illustrate the sequence for implanting a cage according to the invention for lateral entry from the left to right; at the moment of insertion the cage forms a single monolithic body with the keel 111 projecting from the upper contact surface. The cage may be formed as a single body or be obtained by joining together a base cage 12 with one or two adapter plates having a directional guide relief 211 so as to obtain a cage in the form of a single monolithic body.

As can be seen, the entry of the cage (Fig. 16a) is performed from an incorrect position, entering between the vertebrae (for the sake of simpler illustration only the bottom vertebra is shown), and the cage is guided by the keel so as to slide between the vertebrae until it reaches the correct end position (Fig. 16b), i.e. central position in the intervertebral space, which is useful for restoring the correct biomechanics of the vertebral column.

It is therefore clear how the cage according to the invention is able to solve the technical problem of achieving precise and correct intervertebral insertion, both antero-posterior and lateral as well as oblique in the case of a non-optimal insertion point, since hindered by the presence of bone, nerve or vascular structures and/or other anatomical elements which prevent the operator from performing correct positioning at the ideal entry point, without damaging or fracturing the vertebral body and without the need for guide tools or cages with complicated engaging systems, introducing degrees of freedom for the relative movement of cage and tool.

The cage according to the invention is also suitable for use intended for intervertebral prostheses and hip, shoulder or knee replacements and/or for conditioning the anteversion.

Although described in connection with a number of embodiments and a number of preferred examples of implementation of the invention, it is understood that the scope of protection of the present patent is determined solely by the claims below.

## Claims

1. Cage for vertebral arthrodesis operations having a body (10) comprising a first contact surface (10a) for contact with a first vertebral endplate (1) and a second contact surface (10b) for contact with a second vertebral endplate (2), the first and second contact surfaces being arranged opposite each other in a vertical-coronal heightwise direction (Z-Z) of the cage, an entry side (LI) for entry into the intervertebral disc space and a pushing side (LS) opposite to the entry side in a direction of insertion into the intervertebral disc space,
wherein the cage has at least one directional guide relief (11;111;311) formed on at least one of the two said opposite contact surfaces (10a,10b) for contact with a vertebral endplate,
said at least one directional guide relief (11;111;311) is configured and arranged to guide the cage along a predefined movement trajectory for insertion of the cage between the first vertebral endplate (1) and the second vertebral endplate (2) from an entry point as far as a predefined correct end position between the first vertebral endplate and the second vertebral endplate, as a result of insertion of the cage between the vertebral endplates from the entry side by means of action on the pushing side,
**characterized in that** said at least one directional guide relief (11;111;311) comprises a continuous relief which has a rectilinear development inclined in the plane of the contact surface or a mixed development with at least one rectilinear section inclined in the plane of the contact surface, designed to cause a gradual deviation of the cage from a rectilinear insertion trajectory;
and **in that** said at least one directional guide relief (11;111;311) projects from the contact surface of the cage with a height in the vertical-coronal direction (Z-Z) of between 0.5 and 4 mm.

2. Adapter plate (110) for a cage for vertebral arthrodesis operations having a body (10) comprising a first contact surface (10a) and a second contact surface (10b) arranged opposite each other in a coronal heightwise direction (Z-Z) of the cage, an entry side (LI) for entry into the intervertebral disc space and a pushing side (LS) opposite to the entry side in a direction of insertion into the intervertebral disc space,
the adapter plate (110) comprising a plate body which has: a first contact surface (110a) for contact with a vertebral endplate (1) and a second contact surface (110b) for contact with a contact surface (12a,12b) of the cage, the first and second contact surfaces of the plate being opposite each other in a vertical-coronal heightwise direction (Z-Z) of the plate (110); an entry side (LI) for entry into the intervertebral disc space; and a pushing side (LS) opposite to the entry side in a direction of insertion into the intervertebral disc space;
wherein the adapter plate (110) has at least one continuous directional guide relief (211) formed on the first contact surface (110a), which has a rectilinear development inclined in the plane of the first contact surface (110a) or a mixed development with at least one rectilinear section inclined in the plane of the first contact surface (110a); said at least one directional guide relief (211) projecting from the first contact surface of the plate with a height in the vertical-coronal direction (Z-Z) of between 0.5 and 4 mm;
wherein the plate is configured for stable joining together with the cage (12) so as to form therewith a single monolithic body before insertion into the intervertebral disc space and said at least one directional guide relief (211) of the adapter plate (110) is configured and arranged to guide the cage along a predefined movement trajectory for insertion of the cage between the first vertebral endplate (1) and a second vertebral endplate (2), from an entry point as far as a predefined and correct end position between the first vertebral endplate and the second vertebral endplate, as a result of insertion of the cage between the vertebral endplates from the entry side by means of action on the pushing side, wherein said at least one directional guide relief is designed to cause a gradual deviation of the cage from a rectilinear insertion trajectory.

3. Cage according to Claim 1 or adapter plate according to Claim 2, **characterized in that** the at least one directional guide relief (11;111;311) projects from the contact surface of the cage or from the first contact surface (110a) of the plate with a height in the vertical-coronal direction (Z-Z) less than or equal to 2 mm, preferably of between 1 and 2 mm.

4. Cage according to Claim 1 or 3, or adapter plate according to Claim 2 or 3, **characterized in that** it comprises a directional guide relief (311) which has a mixed development with at least one rectilinear section and at least one curvilinear section.

5. Cage or adapter plate according to Claim 4, **characterized in that** the relief (311) with mixed development comprises a first rectilinear section (311a), preferably proximal to the entry side (LI), and a second curvilinear section (311b) connected to the rectilinear section.

6. Cage according to one of Claims 1 and 3-5, or plate according to one of Claims 2 to 5, **characterized in that** the directional guiding relief is uninterrupted, extending over the whole contact surface (10a;10b) from the entry side (LI) to the pushing side (LS); or **in that** the directional guide relief extends over a partial section from the entry side (LI) to the pushing side (LS).

7. Cage according to any one of the preceding Claims 1 and 3-6, or plate according to one of Claims 2 to 6, **characterized in that** at least one of the opposite contact surfaces has an inclination increasing from the entry side (LI) to the pushing side (LS).

8. Cage according to one of the preceding Claims 1 and 3-7, or plate according to one of Claims 2 to 7, **characterized in that** it comprises a plurality of directional reliefs parallel to each other and/or **in that** the at least one directional relief has a cross-sectional shape along a coronal plane which is generally triangular, preferably in the form of an equilateral triangle or rectangle, or quadrangular, rectangular or trapezoidal.

9. Cage according to one of the preceding Claims 1 and 3-8, or plate according to one of Claims 2 to 8, **characterized in that** the at least one directional relief has a width at the point of joining to the cage of between 1 and 4 mm and a width at the point of contact with the vertebral endplate of between 0.5 and 4 mm.

10. Cage according to one of the preceding Claims 1 and 3-9, or plate according to one of Claims 2 to 9, **characterized in that** an angle (α) of inclination of the directional guide relief or of a rectilinear section of the directional guide relief in the plane of the contact surface is between 5° and 45°.

11. Cage according to one of the preceding Claims 1 and 3-10, comprising a cage body (12) and at least one adapter plate according to one of Claims 2 to 10, wherein the cage body (12) comprises a first contact surface (10a) and a second contact surface (12b) arranged opposite each other in a coronal heightwise direction (Z-Z) of the cage, an entry side (LI) for entry into the intervertebral disc space and a pushing side (LS) opposite to the entry side in a direction of insertion into the intervertebral disc space;
Wherein at least one of the first and second contact surfaces (12a,12b) of the cage body (12) is stably joined together with the second contact surface (110b) of an adapter plate of said at least one adapter plate, so as to form a single monolithic body for insertion into the intervertebral disc space, whereby the first contact surface (110a) of the adapter plate forms said at least one contact surface of the cage having formed thereon said at least one directional guide relief.

12. Adapter plate according to one of Claims 2 to 10, or cage according to Claim 11, wherein the adapter plate comprises means for mechanical joining to the body of the cage (12), preferably comprising one or more of the following:
- one or more edges 110c extending in the coronal direction, from an end edge of the plate body;
- one or more fastening systems, in particular of the screw or clip type.

13. Cage according to Claim 11 or 12, wherein the adapter plate covers the whole of the cage surface with which it is in contact or wherein the adapter plate covers only a part of said cage surface; wherein preferably, the plate body is in the form of a strip having a smaller width then the corresponding width of the cage body.

14. Cage according to one of the preceding Claims 1 and 3-13, **characterized in that** it has a transverse dimension greater than the sagittal dimension for latero-lateral insertion.

15. Cage according to one of the preceding Claims 1 and 3-13, **characterized in that** it has a transverse dimension congruous with the sagittal dimension for antero-posterior insertion.

## Patentansprüche

1. Käfig für Wirbel-Arthrodese-Operationen, der einen Körper (10) umfasst, der eine erste Kontaktfläche (10a) für einen Kontakt mit einer ersten Wirbelendplatte (1) und eine zweite Kontaktfläche (10b) für einen Kontakt mit einer zweiten Wirbelendplatte (2), wobei die ersten und zweiten Kontaktflächen einander gegenüberliegend in einer vertikal-koronalen Höhenrichtung (Z-Z) des Käfigs angeordnet sind, eine Eintrittsseite (LI) für einen Eintritt in den Zwischenwirbel-Bandscheibenraum und eine Drückseite (LS) gegenüber der Eintrittsseite in einer Einsteckrichtung in den Zwischenwirbel-Bandscheibenraum aufweist,
wobei der Käfig wenigstens ein Richtungsführungsrelief (11; 111; 311) aufweist, das an wenigstens einer der zwei gegenüberliegenden Kontaktflächen (10a, 10b) für einen Kontakt mit einer Wirbelendplatte ausgebildet ist,
wobei das wenigstens eine Richtungsführungsrelief (11; 111; 311) konfiguriert und angeordnet ist zum Führen des Käfigs entlang einer vordefinierten Bewegungsbahn für das Einstecken des Käfigs zwischen der ersten Wirbelendplatte (1) und der zweiten Wirbelendplatte (2) von einem Eintrittspunkt bis zu einer vordefinierten korrekten Endposition zwischen der ersten Wirbelendplatte und der zweiten Wirbelendplatte, wenn der Käfig zwischen den Wirbelendplatten von der Eintrittsseite mittels einer Betätigung auf der Drückseite eingesteckt wird,
**dadurch gekennzeichnet, dass** das wenigstens eine Richtungsführungsrelief (11; 111, 311) ein kontinuierliches Relief ist, das einen geradlinigen Verlauf, der in der Ebene der Kontaktfläche geneigt ist, oder einen gemischten Verlauf mit wenigstens einem geradlinigen Abschnitt, der in der Ebene der Kontaktfläche geneigt ist, aufweist und ausgebildet ist, um eine allmähliche Abweichung des Käfigs von einer geradlinigen Einsteckbahn zu veranlassen, und
dass das wenigstens eine Richtungsführungsrelief (11; 111; 311) von der Kontaktfläche des Käfigs mit einer Höhe in der vertikal-koronalen Richtung (Z-Z) von zwischen 0,5 und 4 mm vorsteht.

2. Adapterplatte (110) für einen Käfig für Wirbel-Arthrodese-Operationen, der einen Körper (10) umfasst, der eine erste Kontaktfläche (10a) und eine zweite Kontaktfläche (10b), die einander gegenüberliegend in einer koronalen Höhenrichtung (Z-Z) des Käfigs angeordnet sind, eine Eintrittsseite (LI) für einen Eintritt in den Zwischenwirbel-Bandscheibenraum und eine Drückseite (LS) gegenüber der Eintrittsseite in einer Einsteckrichtung in den Zwischenwirbel-Bandscheibenraum aufweist,
wobei die Adapterplatte (110) einen Plattenkörper umfasst, der eine erste Kontaktfläche (110a) für einen Kontakt mit einer Wirbelendplatte (1) und eine zweite Kontaktfläche (110b) für einen Kontakt mit einer Kontaktfläche (12a, 12b) des Käfigs, wobei die ersten und zweiten Kontaktflächen der Platte einander in einer vertikal-koronalen Höhenrichtung (Z-Z) der Platte (110) gegenüberliegen, eine Eintrittsseite (LI) für einen Eintritt in den Zwischenwirbel-Bandscheibenraum und eine Drückseite (LS) gegenüber der Eintrittsseite in einer Einsteckrichtung in den Zwischenwirbel-Bandscheibenraum aufweist,
wobei die Adapterplatte (110) wenigstens ein kontinuierliches Richtungsführungsrelief (211) aufweist, das an der ersten Kontaktfläche (110a) ausgebildet ist und einen geradlinigen Verlauf, der in der Ebene der ersten Kontaktfläche (110a) geneigt ist, oder einen gemischten Verlauf mit wenigstens einem geradlinigen Abschnitt, der in der Ebene der ersten Kontaktfläche (110a) geneigt ist, aufweist, wobei das wenigstens eine Richtungsführungsrelief (211) von der ersten Kontaktfläche der Platte mit einer Höhe in der vertikal-koronalen Richtung (Z-Z) von zwischen 0,5 und 4 mm vorsteht,
wobei die Platte konfiguriert ist für eine stabile Verbindung mit dem Käfig (12), um mit diesem einen einzelnen monolithischen Körper vor dem Einstecken in den Zwischenwirbel-Bandscheibenraum zu bilden, und wobei das wenigstens eine Richtungsführungsrelief (211) der Adapterplatte (110) konfiguriert und angeordnet ist zum Führen des Käfigs entlang einer vordefinierten Bewegungsbahn für das Einstecken des Käfigs zwischen der ersten Wirbelendplatte (1) und einer zweiten Wirbelendplatte (2) von einem Eintrittspunkt bis zu einer vordefinierten und korrekten Endposition zwischen der ersten Wirbelendplatte und der zweiten Wirbelendplatte, wenn der Käfig zwischen den Wirbelendplatten von der Eintrittsseite mittels einer Betätigung auf der Drückseite eingesteckt wird, wobei das wenigstens eine Richtungsführungsrelief ausgebildet ist, um eine allmähliche Abweichung des Käfigs von einer geradlinigen Einsteckbahn zu veranlassen.

3. Käfig nach Anspruch 1 oder Adapterplatte nach Anspruch 2, **dadurch gekennzeichnet, dass** das wenigstens eine Richtungsführungsrelief (11; 111, 311) von der Kontaktfläche des Käfigs oder von der ersten Kontaktfläche (110a) der Platte mit einer Höhe in der vertikal-koronalen Richtung (Z-Z) von weniger oder gleich 2 mm und vorzugsweise zwischen 1 und 2 mm vorsteht.

4. Käfig nach Anspruch 1 oder 3 oder Adapterplatte nach Anspruch 2 oder 3, **dadurch gekennzeichnet, dass** dieser bzw. diese ein Richtungsführungsrelief (311) umfasst, das einen gemischten Verlauf mit wenigstens einem geradlinigen Abschnitt und wenigstens einem gekrümmten Abschnitt aufweist.

5. Käfig oder Adapterplatte nach Anspruch 4, **dadurch gekennzeichnet, dass** das Relief (311) mit einem gemischten Verlauf einen ersten geradlinigen Abschnitt (311a) vorzugsweise in Nachbarschaft zu der Eintrittsseite (LI) und einen zweiten gekrümmten Abschnitt (311b), der mit dem geradlinigen Abschnitt verbunden ist, umfasst.

6. Käfig nach einem der Ansprüche 1 und 3-5 oder Platte nach einem der Ansprüche 2 bis 5, **dadurch gekennzeichnet, dass** das Richtungsführungsrelief ununterbrochen ist und sich über die gesamte Kontaktfläche (10a; 10b) von der Eintrittsseite (LI) zu der Drückseite (LS) erstreckt oder dass sich das Richtungsführungsrelief über einen Teilabschnitt von der Eintrittsseite (LI) zu der Drückseite (LS) erstreckt.

7. Käfig nach einem der vorstehenden Ansprüche 1 und 3 bis 6 oder Platte nach einem der Ansprüche 2 bis 6, **dadurch gekennzeichnet, dass** wenigstens eine der gegenüberliegenden Kontaktflächen eine Neigung aufweist, die sich von der Eintrittsseite (LI) zu der Drückseite (LS) vergrößert.

8. Käfig nach einem der vorstehenden Ansprüche 1 und 3 bis 7 oder Platte nach einem der Ansprüche 2 bis 7, **dadurch gekennzeichnet, dass** dieser bzw. diese eine Vielzahl von parallelen Richtungsreliefs aufweist und/oder dass das wenigstens eine Richtungsrelief eine allgemein dreieckige, vorzugsweise in der Form eines gleichseitigen Dreiecks oder Rechtecks, oder viereckige, rechteckig oder trapezförmig, Querschnittform entlang einer koronalen Ebene aufweist.

9. Käfig nach einem der vorstehenden Ansprüche 1 und 3 bis 8 oder Platte nach einem der Ansprüche 2 bis 8, **dadurch gekennzeichnet, dass** das wenigstens eine Richtungsrelief eine Breite an dem Verbindungspunkt zu dem Käfig zwischen 1 und 4 mm und eine Breite an dem Kontaktpunkt mit der Wirbelendplatte von zwischen 0,5 und 4 mm aufweist.

10. Käfig nach einem der vorstehenden Ansprüche 1 und 3 bis 9 oder Platte nach einem der Ansprüche 2 bis 9, **dadurch gekennzeichnet, dass** der Neigungswinkel (α) des Richtungsführungsreliefs oder eines geradlinigen Abschnitts des Richtungsführungsreliefs in der Ebene der Kontaktfläche zwischen 5° und 45° beträgt.

11. Käfig nach einem der vorstehenden Ansprüche 1 und 3 bis 10, der einen Käfigkörper (12) und wenigstens eine Adapterplatte gemäß einem der Ansprüche 2 bis 10 umfasst, wobei der Käfigkörper (12) eine erste Kontaktfläche (10a) und eine zweite Kontaktfläche (12b), die einander gegenüberliegend in einer koronalen Höhenrichtung (Z-Z) des Käfigs angeordnet sind, eine Eintrittsseite (LI) für einen Eintritt in den Zwischenwirbel-Bandscheibenraum und eine Drückseite (LS) gegenüber der Eintrittsseite in einer Einsteckrichtung in den Zwischenwirbel-Bandscheibenraum aufweist,
wobei wenigstens eine der ersten und zweiten Kontaktflächen (12a, 12b) des Käfigkörpers (12) stabil mit der zweiten Kontaktfläche (110b) einer Adapterplatte der wenigstens einen Adapterplatte verbunden ist, um einen einzelnen monolithischen Körper für das Einstecken in den Zwischenwirbel-Bandscheibenraum zu bilden, wobei die erste Kontaktfläche (110a) der Adapterplatte die wenigstens eine Kontaktfläche des Käfigs mit dem daran ausgebildeten wenigstens einen Richtungsführungsrelief bildet.

12. Adapterplatte nach einem der Ansprüche 2 bis 10 oder Käfig nach Anspruch 11, wobei die Adapterplatte eine Einrichtung für eine mechanische Verbindung mit dem Körper des Käfigs (12) umfasst, die vorzugsweise eines oder mehrere der Folgenden umfasst:
- eine oder mehrere Kanten (110c), die sich in der koronalen Richtung von einer Endkante des Plattenkörpers erstrecken,
- ein oder mehrere Befestigungssysteme, insbesondere des Schraub- oder Cliptyps.

13. Käfig nach Anspruch 11 oder 12, wobei die Adapterplatte die gesamte Käfigfläche, mit der sie in Kontakt ist, bedeckt oder wobei die Adapterplatte nur einen Teil der Käfigfläche bedeckt, wobei der Käfigkörper vorzugsweise die Form eines Streifens mit einer kleineren Breite als die entsprechende Breite des Käfigkörpers aufweist.

14. Käfig nach einem der vorstehenden Ansprüche 1 und 3 bis 13, **dadurch gekennzeichnet, dass** dieser eine transversale Dimension, die größer als die sagittale Dimension ist, für ein latero-laterales Einstecken aufweist

15. Käfig nach einem der vorstehenden Ansprüche 1 und 3 bis 13, **dadurch gekennzeichnet, dass** dieser eine transversale Dimension in Übereinstimmung mit der sagittalen Dimension für ein antero-posteriores Einstecken aufweist.

## Revendications

1. Cage pour les opérations d'arthrodèse vertébrale comportant un corps (10) comprenant une première surface de contact (10a) avec une première plaque terminale vertébrale (1) et une deuxième surface de contact (10b) avec une deuxième plaque terminale vertébrale (2), les première et deuxième surfaces de contact étant disposées opposées l'une à l'autre dans une direction verticale-coronale en hauteur (Z-Z) de la cage, un côté d'entrée (LI) pour l'entrée dans l'espace discal intervertébral et un côté de poussée (LS) opposé au côté d'entrée dans une direction d'insertion dans l'espace discal intervertébral, dans lequel la cage a au moins un relief de guidage directionnel (11, 111; 311) formé sur au moins l'une des deux surfaces de contact opposées (10a, 10b) pour le contact avec une plaque terminale vertébrale,
ledit au moins un relief de guidage directionnel (11, 111; 311) est configuré et agencé pour guider la cage le long d'une trajectoire de mouvement prédéfinie pour l'insertion de la cage entre la première plaque terminale vertébrale (1) et la deuxième plaque terminale vertébrale (2) à partir d'un point d'entrée jusqu'à une position finale correcte prédéfinie entre la première plaque terminale vertébrale et la deuxième plaque terminale vertébrale, à la suite de l'insertion de la cage entre les plaques terminales vertébrales à partir du côté d'entrée au moyen d'une action sur le côté de poussée
**caractérisée en ce que** ledit au moins un relief de guidage directionnel (11, 111; 311) comprend un relief continu qui a un développement rectiligne incliné dans le plan de la surface de contact ou un développement mixte avec au moins une section rectiligne inclinée dans le plan de la surface de contact, conçu pour provoquer une déviation graduelle de la cage suite à une trajectoire d'insertion rectiligne; et **en ce que** ledit au moins un relief de guidage directionnel (11, 111; 311) fait saillie de la surface de contact de la cage avec une hauteur dans la direction verticale-coronale (Z-Z) comprise entre 0,5 et 4 mm.

2. Plaque d'adaptation (110) pour une cage pour des opérations d'arthrodèse vertébrale ayant un corps (10) comprenant une première surface de contact (10a) et une deuxième surface de contact (10b) disposées opposées l'une à l'autre dans une direction coronale en hauteur (Z-Z) de la cage, un côté d'entrée (LI) pour l'entrée dans l'espace discal intervertébral et un côté de poussée (LS) opposé au côté d'entrée dans une direction d'insertion dans l'espace discal intervertébral,
la plaque d'adaptation (110) comprenant un corps de plaque qui a :
une première surface de contact (110a) pour le contact avec une plaque terminale vertébrale (1) et une deuxième surface (110b) pour le contact avec une surface de contact (12a, 12b) de la cage, les première et deuxième surfaces de contact de la plaque étant opposées l'une à l'autre dans une direction verticale-coronale en hauteur (Z-Z) de la plaque (110); un côté d'entrée (LI) pour l'entrée dans l'espace discal intervertébral, et un côté de poussée (LS) opposé au côté d'entrée dans une direction d'insertion dans l'espace discal intervertébral,
dans lequel la plaque d'adaptation (110) présente au moins un relief de guidage directionnel continu (211) formé sur la première surface de contact (110a), qui présente un développement rectiligne incliné dans le plan de la première surface de contact (110a) ou un développement mixte avec au moins une section rectiligne inclinée dans le plan de la première surface de contact (110a); ledit au moins un relief de guidage directionnel (211) faisant saillie à partir de la première surface de contact de la plaque avec une hauteur dans la direction verticale-coronale (Z-Z) comprise entre 0, 5 et 4 mm;
dans laquelle la plaque est configurée pour s'assembler de manière stable avec la cage (12) afin de former avec elle un corps monolithique unique avant insertion dans l'espace discal intervertébral et ledit au moins un relief de guidage directionnel (211) de la plaque d'adaptation (110) est configuré et agencé pour guider la cage le long d'une trajectoire de mouvement prédéfinie pour l'insertion de la cage entre la première plaque terminale vertébrale (1) et une deuxième plaque terminale vertébrale (2), d'un point d'entrée jusqu'à une position finale prédéfinie et correcte entre la première plaque terminale vertébrale et la deuxième plaque terminale vertébrale, à la suite de l'insertion de la cage entre les plaques terminales vertébrales depuis le côté d'entrée au moyen d'une action sur le côté de poussée, dans laquelle ledit au moins un relief de guidage directionnel est conçu pour provoquer une déviation graduelle de la cage suite à une trajectoire d'insertion rectiligne.

3. Cage selon la revendication 1 ou plaque d'adaptation selon la revendication 2, **caractérisée en ce que** le au moins un relief de guidage directionnel (11, 111; 311) fait saillie de la surface de contact de la cage ou de la première surface de contact (110a) de la plaque avec une hauteur dans la direction verticale-coronale (Z-Z) inférieure ou égale à 2 mm, de préférence comprise entre 1 et 2 mm.

4. Cage selon la revendication 1 ou 3, ou plaque d'adaptation selon la revendication 2 ou 3, **caractérisée en ce qu'**elle comprend un relief de guidage directionnel (311) qui présente un développement mixte avec au moins une section rectiligne et au moins une section curviligne.

5. Cage ou plaque d'adaptation selon la revendication 4, **caractérisée en ce que** le relief (311) à développement mixte comprend une première section rectiligne (311a), de préférence proximale au côté d'entrée (LI), et une seconde section curviligne (311b) reliée à la section rectiligne.

6. Cage selon l'une des revendications 1 et 3 à 5, ou plaque selon l'une des revendications 2 à 5, **caractérisée en ce que** le relief de guidage directionnel est ininterrompu, s'étendant sur toute la surface de contact (10a;10b) depuis le côté d'entrée (LI) au côté de poussée (LS); ou **en ce que** le relief de guidage directionnel s'étend sur une section partielle depuis le côté d'entrée (LI) au côté de poussée (LS).

7. Cage selon l'une quelconque des revendications précédentes 1 et 3 à 6, ou plaque selon l'une des revendications 2 à 6, **caractérisée en ce qu'**au moins l'une des surfaces de contact opposées présente une inclinaison croissante du côté d'entrée (LI) au côté de poussée (LS).

8. Cage selon l'une des revendications précédentes 1 et 3 à 7, ou plaque selon l'une des revendications 2 à 7, **caractérisée en ce qu'**elle comprend une pluralité de reliefs directionnels parallèles entre eux et/ou **en ce que** le au moins un relief directionnel a une forme en section transversale selon un plan coronal qui est généralement triangulaire, de préférence en forme d'un triangle équilatéral ou rectangle, ou quadrangulaire, rectangulaire ou trapézoïdale.

9. Cage selon l'une des revendications précédentes 1 et 3 à 8, ou plaque selon l'une des revendications 2 à 8, **caractérisée en ce que** le au moins un relief directionnel a une largeur au point de jonction avec la cage comprise entre 1 et 4 mm et une largeur au point de contact avec la plaque terminale vertébrale comprise entre 0,5 et 4 mm.

10. Cage selon l'une des revendications précédentes 1 et 3 à 9, ou plaque selon l'une des revendications 2 à 9, **caractérisée en ce qu'**un angle (α) d'inclinaison du relief de guidage directionnel ou d'une section rectiligne du relief de guidage directionnel dans le plan de la surface de contact est compris entre 5° et 45°.

11. Cage selon l'une des revendications précédentes 1 et 3 à 10, comprenant un corps de cage (12) et au moins une plaque d'adaptation selon l'une des revendications 2 à 10, dans laquelle le corps de cage (12) comprend une première surface de contact (10a) et une deuxième surface de contact (12b) disposées opposées l'une en face de l'autre dans une direction coronale en hauteur (Z-Z) de la cage, un côté d'entrée (LI) pour l'entrée dans l'espace discal intervertébral et un côté de poussée (LS) opposé au côté d'entrée dans une direction d'insertion dans l'espace discal intervertébral, dans lequel au moins une des premières et deuxièmes surfaces de contact (12a, 12b) du corps de la cage (12) est reliée de manière stable à la deuxième surface de contact (110b) d'une plaque d'adaptation de ladite au moins une plaque d'adaptation, de manière à former un corps monolithique unique à insérer dans l'espace discal intervertébral, la première surface de contact (110a) de la plaque d'adaptation formant ladite au moins une surface de contact de la cage sur laquelle est formé ledit au moins un relief de guidage directionnel.

12. Plaque d'adaptation selon l'une des revendications 2 à 10, ou cage selon la revendication 11, dans laquelle la plaque d'adaptation comprend des moyens d'assemblage mécanique au corps de la cage (12), comprenant de préférence un ou plusieurs des éléments suivants :
- un ou plusieurs bords 110c s'étendant dans la direction coronale, à partir d'un bord d'extrémité du corps de la plaque;
- un ou plusieurs systèmes de fixation, notamment du type vis ou clip.

13. Cage selon la revendication 11 ou 12, dans laquelle la plaque d'adaptation couvre la totalité de la surface de la cage avec laquelle elle est en contact ou dans laquelle la plaque d'adaptation ne couvre qu'une partie de ladite surface de la cage; dans laquelle, de préférence, le corps de la plaque se présente sous la forme d'une bande dont la largeur est inférieure à la largeur correspondante du corps de la cage.

14. Cage selon l'une des revendications précédentes 1 et 3 à 13, **caractérisée en ce qu'**elle présente une dimension transversale supérieure à la dimension sagittale pour une insertion latéro-latérale.

15. Cage selon l'une des revendications précédentes 1 et 3 à 13, **caractérisée en ce qu'**elle a une dimension transversale congruente avec la dimension sagittale pour l'insertion antéro-postérieure.
